# EUROPEAN PATENT APPLICATION

(11) **EP 3 970 605 A1**
(43) Date of publication of application: **23.03.2022**
(21) Application number: 20197493.8
(22) Date of filing: 22.09.2020
(51) Int. Cl.: A61B 5/00

(54) **APPARATUS AND METHOD FOR DETERMINING A SKIN PARAMETER VALUE**

(71) Applicant: Koninklijke Philips N.V., 5656 AG Eindhoven (NL)
(72) Inventor: Boamfa, Marius Iosif, 5656 AE Eindhoven (NL); Verhagen, Rieko, 5656 AE Eindhoven (NL); Van Abeelen, Frank Anton, 5656 AE Eindhoven (NL); Thumma, Kiran Kumar, 5656 AE Eindhoven (NL)
(74) Representative: Philips Intellectual Property & Standards

(57) **Abstract**

According to an aspect, there is provided a method for determining a value of a skin parameter for skin of a subject. The method comprises receiving (510) a first sensor signal that is output by a first light sensor that is arranged to receive light emitted by a first light source after interaction of the emitted light with the skin. The first sensor signal is output while the first light source operates according to a first duty cycle to intermittently emit the light, and the emitted light has a first central wavelength. The method also comprises receiving (520) a first modulation reference signal that is related to the first duty cycle; integrating (530) a first function output signal, resulting from a function of the first sensor signal and the first modulation reference signal, to determine a first integrated value; determining (540) a first number of cycles of the emitted light that are required for the first integrated value to exceed a first threshold value; and determining (550) the value of the skin parameter based on the determined first number of cycles.

## Description

### FIELD OF THE INVENTION

This disclosure relates to an apparatus and method for determining a value of a skin parameter.

### BACKGROUND OF THE INVENTION

Many treatment devices require the determination of a skin parameter value before, during or after use. Skin parameter values used by treatment devices can include skin tone, skin melanin index, and other skin parameters that relate to skin colour, skin patterns or skin features (e.g. the presence of hair, moles, scars, acne, etc.). Examples of treatment devices that can require determination of a skin parameter value include devices for the removal of unwanted hairs using various techniques such as laser and light therapies (known as photoepilation or Intense Pulsed Light, IPL). Further examples include devices for treating acne and skin lesions.

IPL technology is a popular solution for many applications such as, but not limited to, photoepilation, lesion treatment, photo-rejuvenation, in-home personal care, professional personal care and medical settings. For photoepilation in a home environment, IPL photoepilation devices apply broad-spectrum light to the surface of the skin, targeting the melanin in the hair and hair follicles. Hair and the follicle (that are in their anagen phase of the growth cycle) absorbs this energy and goes into its resting phase. This prevents the regrowth of hair. For effective use of this IPL technology for hair removal (e.g. to minimise damage, thermal injury/burns or irritation to the skin), the energy setting of the IPL photoepilation device can be adapted based on the skin tone of the skin. Some IPL photoepilation devices, e.g. the Philips Lumea Prestige, can detect the skin tone before and during a treatment, and select an appropriate energy setting. The skin tone can be detected and categorised into one of, e.g. six, different types. The skin types 1 to 6 can be broadly labelled as: 'white', 'beige', 'light brown', 'medium brown', 'dark brown' and 'brownish black and darker'. Typically IPL photoepilation devices should not be used with darker skin as the skin will absorb energy in the light pulse rather than the hair or follicle. In that case, if a skin tone of e.g. brownish black and darker, is detected, the device should not trigger a flash.

A current method of skin type detection in IPL devices uses reflectance spectroscopy, for example as described in "A portable reflectometer for a rapid quantification of cutaneous haemoglobin and melanin" by Feather J.W., Ellis D.J., and Leslie G., Phys. Med. Biol. 1988, 33, 711-722. In this technique, the ratio between two reflected wavelengths (red and near infrared - melanin has a higher absorption coefficient at these two wavelengths compared to water and haemoglobin) are used to compute a melanin index, which is then used to compute skin type. One type of skin tone sensor that is currently used in IPL devices makes use of two light emitting diodes (LEDs) that operate at two distinct wavelengths (640 nanometres (nm) and 870 nm central wavelengths respectively). The light of these two LEDs is emitted towards the skin and a detector measures the reflected light, resulting in detector signals S₁ and S₂ for the two LEDs respectively. Based on the levels of skin reflectivity for the two wavelengths a skin tone can be computed, with skin tone being a function of the ratio S₁/S₂.

However, the reflected energy signals are affected by temperature, ambient light, and the waveguide of the IPL device. An alternative to this method is to use smartphone camera images of a skin region to compute skin tone. This is also very challenging due to specular reflectivity of skin, variance in illumination conditions, and variance in image sensor characteristics. Furthermore, the image measurement may require use of a colour calibration card.

As a result of these and other factors, methods that rely on reflectance spectroscopy to determine skin tone and other skin parameter values have a large tolerance. In the case of an IPL treatment device, a large tolerance can lead to the device not blocking dark skin types when it should, or blocking a significant portion of users that should be able to use the device. Therefore, improvements are desired for determining a skin parameter such as skin tone.

### SUMMARY OF THE INVENTION

According to a first specific aspect, there is provided a method for determining a value of a skin parameter for skin of a subject. The method comprises: (i) receiving a first sensor signal that is output by a first light sensor that is arranged to receive light emitted by a first light source after interaction of the emitted light with the skin, wherein the first sensor signal is output while the first light source operates according to a first duty cycle to intermittently emit the light, and wherein the emitted light has a first central wavelength; (ii) receiving a first modulation reference signal that is related to the first duty cycle; (iii) integrating a first function output signal, resulting from a function of the first sensor signal and the first modulation reference signal, to determine a first integrated value; (iv) determining a first number of cycles of the emitted light that are required for the first integrated value to exceed a first threshold value; and (v) determining the value of the skin parameter based on the determined first number of cycles. Thus, by intermittently emitting the light and integrating a function of the sensor signal and a modulation reference signal that is related to the duty cycle of the light emissions, the method provides that the contribution of ambient light to the sensor signal can be removed or substantially reduced in a simple way to enable a more reliable skin parameter value to be determined.

In some embodiments, the function of the first sensor signal and the first modulation reference signal comprises a product of the first sensor signal and the first modulation reference signal.

In some embodiments, the first modulation reference signal has a modulation period corresponding to a modulation period of the first duty cycle. This has the advantage that a simple function of the first modulation reference signal and the first sensor signal can be used.

In some embodiments, the first modulation reference signal and the function of the first sensor signal and the first modulation reference signal result in the first function output signal corresponding to a first multiple of the first sensor signal for parts of the first duty cycle in which the first light source is emitting light, and corresponding to the inverse of the first multiple of the first sensor signal for parts of the first duty cycle in which the first light source is not emitting light. In this way, when the first function output signal is integrated the inverted parts of the first sensor signal corresponding to ambient light will compensate the contribution of ambient light to the parts of the first sensor signal corresponding to when the first light source was emitting light.

In some embodiments, the first sensor signal is an analog signal output by the first light sensor. In some of these embodiments, the step of integrating comprises integrating the first function output signal in the analog domain. This has the advantage that the integration will not include noise introduced as part of an analog-to-digital conversion of the function or first sensor signal.

In some embodiments, the step of integrating comprises inputting the first function output signal, resulting from the function of the first sensor signal and the first modulation reference signal, into one or more analog electronics integrators. In some of these embodiments, the first integrated value is an electrical voltage across the one or more analog electronics integrators.

In some embodiments, the one or more analog electronics integrators are one or more capacitors, and the first threshold value is a value for the electrical voltage across the one or more capacitors. This has the advantage that simple analog electronics components can be used to determine the skin parameter value.

In alternative embodiments, the step of integrating comprises integrating the first function output signal, resulting from the function of the first sensor signal and the first modulation reference signal, in the digital domain. These embodiments have the advantage that more complex signal processing techniques can be used, for example to reduce noise in the first sensor signal.

In some embodiments, the first central wavelength is 640 nm (nanometres), 870 nm, a wavelength corresponding to visible light, a wavelength corresponding to infrared light, a wavelength in the range of 600 nm to 700 nm, or a wavelength in the range of 800 nm to 900 nm.

In some embodiments, the skin parameter is skin tone.

In some embodiments, the first duty cycle is 50%. These embodiments have the advantage that the signal processing (e.g. in the analog domain or the digital domain) is simplified.

In some embodiments, the method further comprises: receiving a second sensor signal that is output by the first light sensor or a second light sensor that is arranged to receive light emitted by the first light source or a second light source after interaction of the emitted light with the skin, wherein the second sensor signal is output while the first light source or the second light source operates according to a second duty cycle to intermittently emit light having a second central wavelength different from the first central wavelength; receiving a second modulation reference signal that is related to the second duty cycle; integrating a second function output signal, resulting from a function of the second sensor signal and the second modulation reference signal, to determine a second integrated value; and determining a second number of cycles of the emitted light having the second central wavelength that are required for the second integrated value to exceed a second threshold value; wherein the step of determining the value of the skin parameter comprises determining the value of the skin parameter based on a function of the determined first number of cycles and the determined second number of cycles.

In some of these embodiments, the function of the determined first number of cycles and the determined second number of cycles is a ratio of the determined first number of cycles and the determined second number of cycles.

In some embodiments, the second central wavelength is 640 nm (nanometres), 870 nm, a wavelength corresponding to visible light, a wavelength corresponding to infrared light, a wavelength in the range of 600 nm to 700 nm, or a wavelength in the range of 800 nm to 900 nm.

In some embodiments, the skin parameter is skin tone.

According to a second aspect, there is provided a computer program product comprising a computer readable medium having computer readable code embodied therein, the computer readable code being configured such that, on execution by a suitable computer or processor, the computer or processor is caused to perform according to the first aspect or any embodiment thereof.

According to a third aspect, there is provided an apparatus for determining a value of a skin parameter for skin of a subject. The apparatus is configured to: (i) receive a first sensor signal that is output by a first light sensor that is arranged to receive light emitted by a first light source after interaction of the emitted light with the skin, wherein the first sensor signal is output while the first light source operates according to a first duty cycle to intermittently emit the light, and wherein the emitted light has a first central wavelength; (ii) receive a first modulation reference signal that is related to the first duty cycle; (iii) integrate a first function output signal, resulting from a function of the first sensor signal and the first modulation reference signal, to determine a first integrated value; (iv) determine a first number of cycles of the emitted light that are required for the first integrated value to exceed a first threshold value; and (v) determine the value of the skin parameter based on the determined first number of cycles. Thus, by intermittently emitting the light and integrating a function of the sensor signal and a modulation reference signal that is related to the duty cycle of the light emissions, the method provides that the contribution of ambient light to the sensor signal can be removed or substantially reduced in a simple way to enable a more reliable skin parameter value to be determined.

In some embodiments, the function of the first sensor signal and the first modulation reference signal comprises a product of the first sensor signal and the first modulation reference signal.

In some embodiments, the first modulation reference signal has a modulation period corresponding to a modulation period of the first duty cycle. This has the advantage that a simple function of the first modulation reference signal and the first sensor signal can be used.

In some embodiments, the first modulation reference signal and the function of the first sensor signal and the first modulation reference signal result in the first function output signal corresponding to a first multiple of the first sensor signal for parts of the first duty cycle in which the first light source is emitting light, and corresponding to the inverse of the first multiple of the first sensor signal for parts of the first duty cycle in which the first light source is not emitting light. In this way, when the first function output signal is integrated the inverted parts of the first sensor signal corresponding to ambient light will compensate the contribution of ambient light to the parts of the first sensor signal corresponding to when the first light source was emitting light.

In some embodiments, the first sensor signal is an analog signal output by the first light sensor. In some of these embodiments, the apparatus is configured to integrate the first function output signal, resulting from the function of the first sensor signal and the first modulation reference signal, by integrating in the analog domain. This has the advantage that the integration will not include noise introduced as part of an analog-to-digital conversion of the function or first sensor signal.

In some embodiments, the apparatus is configured to integrate the first function output signal, resulting from the function of the first sensor signal and the first modulation reference signal, by inputting the first function output signal into one or more analog electronics integrators.

In some embodiments, the first integrated value is an electrical voltage across the one or more analog electronics integrators.

In some embodiments, the one or more analog electronics integrators are one or more capacitors, and the first threshold value is a value for electrical voltage across the one or more capacitors. This has the advantage that simple analog electronics components can be used to determine the skin parameter value.

In alternative embodiments, the apparatus is configured to integrate the first function output signal, resulting from the function of the first sensor signal and the first modulation reference signal, by integrating in the digital domain. These embodiments have the advantage that more complex signal processing techniques can be used, for example to reduce noise in the first sensor signal.

In some embodiments, the first central wavelength is 640 nm (nanometres), 870 nm, a wavelength corresponding to visible light, a wavelength corresponding to infrared light, a wavelength in the range of 600 nm to 700 nm, or a wavelength in the range of 800 nm to 900 nm.

In some embodiments, the skin parameter is skin tone.

In some embodiments, the first duty cycle is 50%. These embodiments have the advantage that the signal processing (e.g. in the analog domain or the digital domain) is simplified.

In some embodiments, the apparatus is further configured to: (i) receive a second sensor signal that is output by the first light sensor or a second light sensor that is arranged to receive light emitted by the first light source or a second light source after interaction of the emitted light with the skin, wherein the second sensor signal is output while the first light source or the second light source operates according to a second duty cycle to intermittently emit light having a second central wavelength different from the first central wavelength; (ii) receive a second modulation reference signal that is related to the second duty cycle; (iii) integrate a second function output signal, resulting from a function of the second sensor signal and the second modulation reference signal, to determine a second integrated value; and (iv) determine a second number of cycles of the light having the second central wavelength that are required for the second integrated value to exceed a second threshold value; wherein the apparatus is configured to determine the value of the skin parameter based on a function of the determined first number of cycles and the determined second number of cycles.

In some of these embodiments, the function of the determined first number of cycles and the determined second number of cycles is a ratio of the determined first number of cycles and the determined second number of cycles.

In some embodiments, the second central wavelength is 640 nm (nanometres), 870 nm, a wavelength corresponding to visible light, a wavelength corresponding to infrared light, a wavelength in the range of 600 nm to 700 nm, or a wavelength in the range of 800 nm to 900 nm.

In some embodiments, the skin parameter is skin tone.

According to a fourth aspect, there is provided a system comprising: an apparatus for determining a value of a skin parameter for skin of a subject according to the third aspect or any embodiment thereof; the first light source configured to operate according to the first duty cycle to intermittently emit the light having the first central wavelength; and the first light sensor configured to output the first sensor signal.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary treatment device with which the invention can be used;
Fig. 2 is a block diagram of an exemplary system comprising a light source(s), a light sensor(s) and an apparatus according to various embodiments;
Fig. 3 is a schematic showing an exemplary embodiment of the invention;
Fig. 4 is a flow chart illustrating a method for determining a skin tone according to various embodiments of the invention; and
Fig. 5 is a flow chart illustrating an exemplary method for determining a value of a skin parameter for skin of a subject.

### DETAILED DESCRIPTION OF THE EMBODIMENTS

As noted above, the determination of a value of a skin parameter using spectroscopic techniques can result in skin parameter values having a large tolerance. A significant contributing factor to these large tolerances is ambient light measured by the light sensor. The present disclosure provides for a method and apparatus for determining a skin parameter value that is less sensitive to ambient light and can work well for all skin tones.

In some embodiments, the skin parameter is skin tone or melanin index. However, it will be appreciated that the determined skin parameter does not have to be skin tone. Indeed, the skin parameter can be any skin parameter that can be determined from light reflected or otherwise received from skin. For example, the determined skin parameter could be the skin colour, or the presence or absence of skin patterns or skin features (e.g. the presence of (dark) hairs, moles, scars, acne, etc.).

The techniques can be implemented by a treatment device that may make use of the determined skin parameter during the operation or use of the treatment device. In an exemplary embodiment the treatment device is one that uses light pulses to remove hair and/or reduce hair growth. However, the techniques described herein are not restricted to use by treatment devices that use light pulses to remove hair and/or reduce hair growth, and they can be used by other types of treatment devices. Moreover, it will also be appreciated that the skin parameter does not have to be used by a treatment device or for a purpose associated with a treatment device. For example the method disclosed herein can be used to determine a skin parameter (e.g. skin type and/or melanin index) that can be used to recommend a skin product (e.g. makeup or concealer), and/or assess a pigment disorder.

The present disclosure provides a light source, such as one or more LED units, that is operated according to a first duty cycle to intermittently emit light, i.e. the emitted light is modulated in the time domain. In a preferred embodiment the modulation is a square on/off modulation (i.e. the light source emits light in the 'on' phase, and the light source does not emit light in the 'off phase), but other modulations are possible such as sinusoidal, triangular, saw-tooth or any other periodic signal. In a preferred embodiment the modulation of the light varies between 0 and 1 (on and off states), but other embodiments are possible. The duty cycle (i.e. the ratio of the duration of the on and off states) of the modulated light is preferably 50%, but can take other values, e.g. 30%, 40%, 60%, 70%, etc. After interacting with skin (e.g. being reflected by the skin or passing through the skin), the modulated light is incident on a light sensor (which is also referred to as a light detector). The light incident on the sensor will be a combination of the modulated light that has interacted with the skin and ambient (unmodulated) light, and the signal generated by the light sensor will further include detector noise.

According to the present disclosure, the contribution of the non-modulated, ambient light component can be greatly reduced determining a function of the detector signal and a modulation reference signal and subsequently integrating the function in the time domain. In a preferred embodiment, the modulation reference signal varies between -1 and +1 states, but alternative embodiments are possible in which the modulation reference signal is varied between -X and +X, or -X and +Y states. In a preferred embodiment, the modulation reference signal varies between the +1 (or +X) and -1 (or -X) states in line with the duty cycle of the emitted light. In other words, the modulation period of the modulation reference signal is preferably the same as the modulation period of the duty cycle, and in determining the function, the modulation reference signal is 'in phase' with the duty cycle, meaning that 'on' periods of the duty cycle correspond to +1 (or +X) periods of the modulation reference signal, and 'off' periods of the duty cycle correspond to -1 (or -X) periods of the modulation reference signal. However, alternative embodiments are possible. In some embodiments, the function of the detector signal and the modulation reference signal is the product of the detector signal and the modulation reference signal. In some embodiments, the detector signal may be multiplied by the modulation reference signal and a further correction factor, and subsequently integrated. The integration of the function of the detector signal and the modulation reference greatly reduces the contribution of the non-modulated ambient light.

It is proposed herein to determine the number of modulation cycles of the emitted light needed for the integrated value to reach a pre-chosen threshold level. The number of modulation cycles, *N*, is proportional to the reflectivity of the skin under investigation. In an exemplary embodiment, the skin tone of the skin is determined using a function of the ratio *N*₂/*N*₁, where *N*₁ and *N*₂ are the number of modulation cycles determined for two light sources of different central wavelengths. Darker skin types have lower reflectivity and therefore the integration time to reach the threshold level will be longer than for lighter skin types. The relatively long integration time improves the signal to noise ratio of the detector signal. Thus, the method and apparatus disclosed herein greatly reduce the effects of ambient light and detector noise. The disclosed techniques are also more sensitive to low levels of reflected or scattered light, and can therefore be used to determine skin tone and other skin parameters for a wider range of skin tones than current methods and devices.

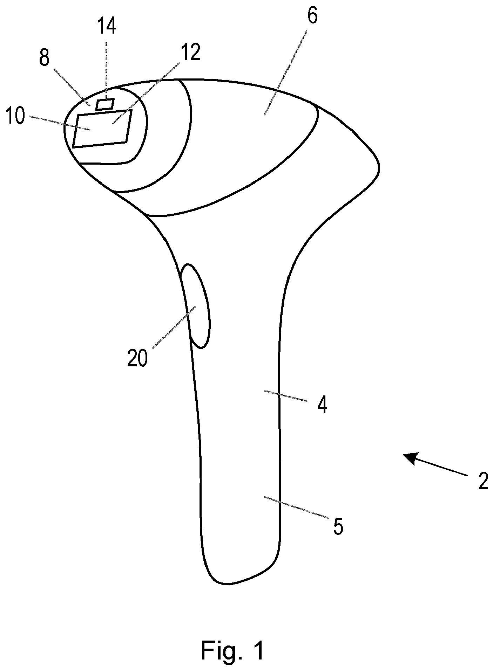

Fig. 1 is an illustration of an exemplary treatment device 2 that can be used to apply a light pulse to an area of skin. It will be appreciated that the treatment device 2 in Fig. 1 is merely presented as an example of a treatment device 2 that the invention can be used with, and the treatment device 2 is not limited to the form shown in Fig. 1 or to being a handheld treatment device. As noted above, the invention is likewise not limited to being implemented in or with a treatment device 2, and in some embodiments the invention can be implemented in an apparatus that is provided for the purpose of determining skin parameter values.

The treatment device 2 in Fig. 1 is for use on a body of a subject (e.g. a person or an animal), and is to be held in one or both hands of a user during use. The treatment device 2 is to perform some treatment operation to hairs on the body of the subject using one or more light pulses when the treatment device 2 is in contact with a body part of the subject.

The exemplary treatment device 2 comprises a housing 4 that includes at least a handle portion 5 and a head portion 6. The handle portion 5 is shaped to enable the user to hold the treatment device 2 with one hand. The head portion 6 has a head end 8that is to be placed into contact with the subject in order for the treatment operation to be performed on the body or skin of the subject at the position that the head end 8 is in contact with the body or skin.

The treatment device 2 is for performing a treatment operation using light pulses. Thus, in Fig. 1 the head end 8 comprises an aperture 10 that is arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10.

The one or more light sources 12 can generate light pulses of any suitable or desired wavelength (or range of wavelengths) and/or intensities. The one or more light sources 12 are configured to provide pulses of light. That is, the light source(s) 12 are configured to generate light at a high intensity for a short duration (e.g. less than 1 second). The intensity of the light pulse should be high enough to effect the treatment operation on the skin or body part adjacent the aperture 10.

In addition to the one or more light sources 12 for effecting the light-based treatment operation, the device also comprises a skin parameter sensor 14 for determining a value of a skin parameter for skin of a subject, such as skin tone. The skin parameter sensor 14 comprises one or more light source(s) and one or more light sensor(s). An apparatus is provided, either within the treatment device 2, or separate from the treatment device 2, for determining a skin parameter value using an output signal from the light sensor(s).

The illustrated treatment device 2 also includes a user control 20 that can be operated by the user to activate the treatment device 2 so that the required treatment operation is performed on the body of the subject (e.g. the generation of one or more light pulses by the one or more light source(s) 12). The user control 20 may be in the form of a switch, a button, a touch pad, etc.

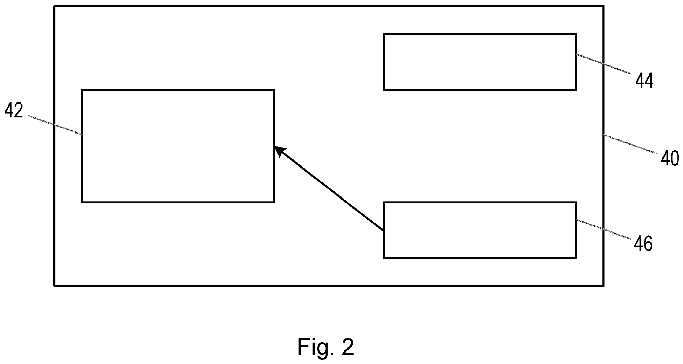

Fig. 2 is a block diagram of an exemplary system 40 for determining a skin parameter value according to the techniques described herein. The skin parameter sensor 14 in the treatment device 2 of Fig. 1 can be an example of system 40. The system 40 comprises an apparatus 42 for determining a value of a skin parameter for skin of a subject, one or more light sources 44 for emitting light and one or more light sensors 46 for measuring incident light. The apparatus 42 can be a device or set of components dedicated to determining a skin parameter value, but in other embodiments the apparatus 42 can be a suitably programmed or suitably configured general purpose device, such as a smart phone, smart watch, tablet, personal digital assistant (PDA), laptop, desktop computer, remote server, smart mirror, etc.

The one or more light sources 44 are provided for illuminating the skin area of interest (i.e. the skin area for which a skin parameter value is to be determined). The light source(s) 44 for illuminating the skin can be any suitable light source, e.g. one or more LEDs, and can emit light at respective central wavelength(s), or in respective particular ranges of wavelengths. In an exemplary embodiment for example where skin tone is to be determined, the light source(s) 44 comprise two LEDs operated at two distinct central wavelengths. For example, LED 1 may have a central wavelength of 640 nm and LED 2 may have a central wavelength of 870 nm.

When the system 40 is in use, the light source(s) 44 are configured to emit light towards the skin of a subject. The light sensor(s) 46 are configured to measure the light emitted by the light source(s) after the emitted light has interacted with the skin. For example, the light may be reflected, transmitted or scattered by the skin. There may be a single light sensor 46 for detecting light from the one or more light source(s) 44. In alternative embodiments, there may be more than one light sensor 46, e.g. a respective light sensor 46 may be provided for each light source 44. Each light sensor 46 is responsive to incident light, and each light sensor 46 outputs a sensor signal, that is provided to the apparatus 42.

The apparatus 42 receives the sensor signal(s) and determines a skin parameter value using the sensor signal(s) as described further below. In some embodiments, the apparatus 42 may determine the skin parameter value in the analog domain using analog electrical circuitry and components. In other embodiments the apparatus 42 may determine the skin parameter value in the digital domain, and thus the apparatus 42 may comprise a processing unit that generally controls the operation of the apparatus 42 and enables the apparatus 42 to perform some or all of the method and techniques described herein. The processing unit 48 can be implemented in numerous ways known to the person skilled in the art, with software and/or hardware, to perform the various functions described herein.

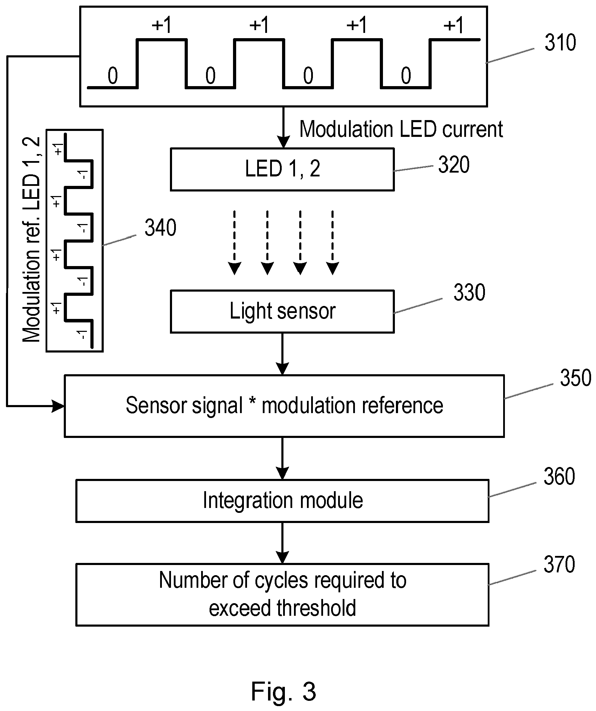

Fig. 3 shows an exemplary embodiment of the present disclosure. In Fig. 3 there are one or two LEDs 320, e.g. LED 1 and optionally also LED 2, and the steps shown are applicable to one or both LEDs 320. In the case of two LEDs 320, the central wavelength of the light emitted by LED 1, *λ*₁, can be distinct from the central wavelength emitted by LED 2, *λ*₂.

The LED(s) 320 are driven by an LED current 310. As illustrated in Fig. 3, to cause the LED(s) 320 to intermittently emit light, the LED current 310 is modulated in the time domain with a square on/off modulation and a duty cycle of 50%. Thus, the light emitted by the LED 320 is also modulated in the time domain with a square on/off modulation and a 50% duty cycle. As noted above, other modulations of the LED 320 are possible including sinusoidal, triangular, saw-tooth, or any other periodic signal, and the driving current 310 can be adapted accordingly. Furthermore, the duty cycle can take any other value.

After interacting with skin (e.g. reflecting, scattering, etc.), the emitted light is incident on a light sensor 330 which produces a sensor signal. The sensor signal may be formed by the current generated as light is incident on the light sensor. The light sensor 330 operates the measure of the incident light across multiple cycles of operation of the LED(s) 320, and thus the light sensor 330 generates the sensor signal during periods when the LED 320 is emitting light and periods when the LED 320 is not emitting light. The light incident on the light sensor 330 when the LED 320 is emitting light comprises the light that has interacted with the skin, and also ambient light. The light incident on the light sensor 330 when the LED 320 is not emitting light just comprises the ambient light.

The sensor signal is subsequently multiplied by a modulation reference signal 340. The modulation reference signal is a signal that is used in combination with the sensor signal to reduce the influence of ambient light on the determined skin parameter value. Thus, in the exemplary embodiment of Fig. 3, the modulation reference signal 340 is a square wave signal that varies between -1 and +1 states/values and has the same period as the modulated light emitted by the relevant LED(s) 320. The modulation reference signal is in phase with the sensor signal, which means that the parts of the sensor signal corresponding to when light was emitted by the LED 320 are multiplied by the +1 or +X part of the modulation reference signal. This also means that the parts of the sensor signal corresponding to when light is not emitted by the LED 320 are multiplied by the -1 or -X part of the modulation reference signal, which acts to reduce or remove the influence of the ambient light from the subsequent analysis of the sensor signal. However, other embodiments are also possible. For example, the modulation reference signal 340 may vary between -X and +X, or -X and +Y states. It is further noted that alternative functional operations can be applied to the sensor signal and the modulation reference signal other than multiplication of the two signals 350. As noted above, this could be a scaling or other functional operation to account for different modulation functions to those in the exemplary embodiment shown in Fig. 3.

In a subsequent step, the product of the sensor signal and the modulation reference signal is integrated in an integration module 360 to obtain an integrated value. The use of an integration step enables signals that are below a detection limit (e.g. due to low levels of reflected light) to be integrated to a level above a detection limit. This enables darker skin tones to be measured in the presence of high levels of background (ambient) light. In a preferred embodiment, the integration is done in the analog domain by an analog electronics integrator. In these embodiments, the integrated value is an electrical voltage across the analog electronics integrator. For example, the integration can be performed by an analog electronic device such as, but not limited to, a capacitor, which accumulates electrical charge as the function of the sensor signal and modulation reference signal is applied to the capacitor. The integrated value is an electrical voltage across the capacitor. Analog integration is beneficial because it avoids the need to convert the sensor signal (or a signal derived from the sensor signal) into the digital domain, which would introduce noise. In an alternative embodiment, the integration step can be performed in the digital domain. The use of digital integration may be beneficial in embodiments that require mathematical corrections to account for modulation functions other than those shown in the exemplary embodiment shown in Fig. 3 (i.e. different to square on/off modulation with a 50% duty cycle). However, it is noted that digital integration will introduce further noise.

In the final step of Fig. 3, the number of modulation cycles 370 needed for the integrated value to exceed a threshold value is determined. That is, as the LED 320 is intermittently operated and the sensor signal obtained, the function of the sensor signal is continuously integrated (e.g. by inputting the product signal into an analog electronics integrator, such as a capacitor). The integrated value therefore increases over the course of several modulation (on/off) cycles of the LED 320. The contribution to the integrated value by each cycle is proportional to the reflectivity of the skin. Thus, the number of modulation cycles 370 needed for the integrated value to exceed the threshold value is also proportional to the reflectivity of the skin. In some embodiments, the threshold value is a value for electrical voltage across the analog electronics integrator (e.g. capacitor).

In embodiments where the skin parameter is skin tone and two LEDs 320 are used, the number of cycles 370 required to exceed a threshold value can be denoted *N*₁ for LED 1 and *N*₂ for LED 2. The skin tone can be given as a function of the ratio *N*₂/*N*₁*.* As noted above, darker skin types have lower reflectivity than lighter skin types, and therefore darker skin will require longer integration times (i.e. more modulation cycles) for the integrated value to exceed the threshold value. This relatively long integration time, and the removal or reduction of the ambient light through the use of the modulation reference signal and the subsequent integration, improves the signal to noise ratio. Thus, the present invention provides a skin tone sensor that is both insensitive to ambient light and can work optimally for all skin tones.

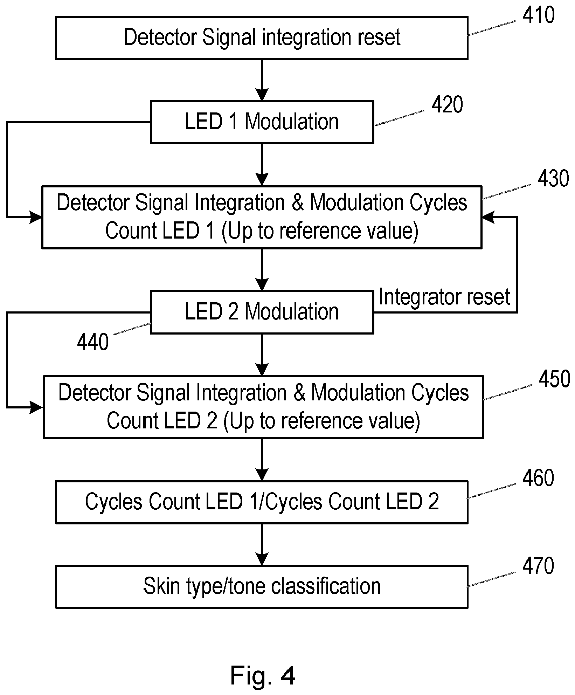

The flow chart in Fig. 4 illustrates a method for determining a skin tone according to various embodiments of the invention. The method includes the modulation of two LED units, LED 1 and LED 2. In step 410 an integration signal is reset (e.g. set to 0). In step 420, a modulated light signal is emitted by LED 1 and is incident on the skin of a subject. In step 430, the light emitted by LED 1 that has interacted with the skin is incident on a light sensor and the corresponding detector signal (e.g. sensor signal) is multiplied by a modulation reference signal. This combined signal is integrated over the course of a number of modulation cycles, and the number of modulation cycles required for the integrated value to exceed a threshold value is determined. In steps 440 and 450, steps 420 and 430 are repeated for LED 2 (which emits light of a different central wavelength to LED 1). The ratio of the determined number of cycles for LED 1 and LED 2 is obtained in step 460. Finally, this ratio is used in step 470 to determine the skin tone classification.

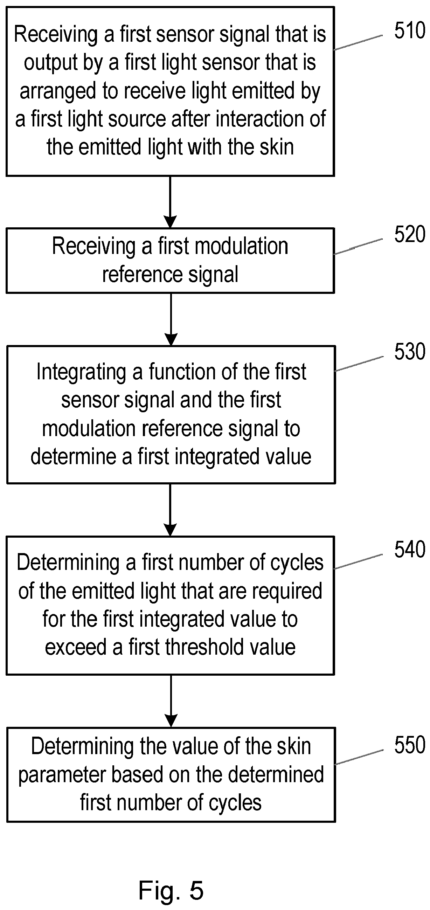

The flow chart in Fig. 5 illustrates an exemplary method according to the techniques described herein for determining a value of a skin parameter for skin of a subject. This method can be performed by the apparatus 42 described above. The method begins at step 510 with receiving a first sensor signal that is output by a first light sensor. The first light sensor is arranged to receive light emitted by a first light source after interaction of the emitted light with the skin. The first sensor signal received in step 510 is output while the first light source operates according to a first duty cycle to intermittently emit light with a first central wavelength. In some embodiments, the first light source is a LED. The first central wavelength can be 640 nm (nanometres), 870 nm, a wavelength corresponding to visible light, a wavelength corresponding to infrared light, a wavelength in the range of 600 nm to 700 nm or a wavelength in the range of 800 nm to 900 nm. In some embodiments, the first duty cycle is 50%. The first sensor signal can be an analog signal output by the first light sensor. In some of these embodiments, the first sensor signal is a current signal.

In step 520 a first modulation reference signal is received that is related to the first duty cycle. The first modulation reference signal preferably has a modulation period corresponding to the modulation period of the intermittent emission of light by the first light source.

In step 530 a function of the first sensor signal and the first modulation reference signal is integrated to determine a first integrated value. In some embodiments, the function comprises a product of the first sensor signal and the first modulation reference signal. In embodiments where the first sensor signal is an analog signal, the step of integrating can be performed in the analog domain. In some of these embodiments, step 530 can comprise inputting a first function output signal, resulting from the function of the first sensor signal and the first modulation reference signal, into one or more analog electronics integrators. In these embodiments, the first integrated value will be the electrical voltage across the one or more analog electronics integrators. In some embodiments, the one or more analog electronics integrators are one or more capacitors. In an alternative embodiment, the step of integrating is in the digital domain.

In step 540 a first number of cycles of the emitted light is determined that is required for the first integrated value to exceed a first threshold value. In some embodiments, in which step 530 is in the analog domain, the first threshold value can be a value for electrical voltage across the one or more analog electronics integrators (e.g. capacitor(s)).

In step 550 the value of the skin parameter can be determined based on the determined first number of cycles.

In some embodiments, step 550 further comprises determining the value of the skin parameter based on a function of the determined first number of cycles and a determined second number of cycles. In these embodiments, the determined second number of cycles is obtained by repeating steps 510-540 for a second sensor signal that is output while light having a second central wavelength is intermittently emitted according to a second duty cycle on to the skin. The second sensor signal may be output by the first light sensor or by a second light sensor. The light having the second central wavelength can be emitted by the first light source or by a second light source. The second light source may be an LED. The second central wavelength is different from the first central wavelength. The second central wavelength may be any of 640 nm, 870 nm, a wavelength corresponding to visible light, a wavelength corresponding to infrared light, a wavelength in the range of 600 nm to 700 nm, or a wavelength in the range of 800 nm to 900 nm. In some embodiments (e.g. when the skin parameter is skin tone), one of the first central wavelength and the second central wavelength is a wavelength corresponding to visible light (e.g. 640 nm or a wavelength in the range of 600 nm to 700 nm), and the other one of the first central wavelength and the second central wavelength is a wavelength corresponding to infrared light (e.g. 870 nm or a wavelength in the range of 800 nm to 900 nm). In preferred embodiments, the second duty cycle is 50%.

In these embodiments, the step 550 of Fig. 5 further comprises receiving a second modulation reference signal that is related to the second duty cycle. The second modulation reference signal preferably has a modulation period corresponding to the modulation period of the intermittent emission of light having the second central wavelength.

In these embodiments, step 550 can further comprise integrating a function of the second sensor signal and the second modulation reference signal to determine a second integrated value. In some of these embodiments, the function comprises a product of the second sensor signal and the second modulation reference signal. In embodiments where the second sensor signal is an analog signal, the step of integrating can be performed in the analog domain. In some of these embodiments, the step of integrating can comprise inputting a second function output signal, resulting from the function of the second sensor signal and the second modulation reference signal, into one or more analog electronics integrators. The second integrated value will be the electrical voltage across the one or more analog electronics integrators. In some embodiments, the one or more analog electronics integrators are one or more capacitors. In an alternative embodiment, the step of integrating is performed in the digital domain.

In these embodiments, step 550 can further comprise determining the aforementioned second number of cycles as the number of cycles of the light having the second central wavelength that are required for the second integrated value to exceed a second threshold value. In embodiments in which the integration is performed in the analog domain, the second threshold value can be a value for the electrical voltage across the one or more analog electronics integrators (e.g. capacitor(s)). The second threshold value can be the same value or a different value to the first threshold value.

In some of these embodiments, the function of the determined first number of cycles and the determined second number of cycles is, or includes, a ratio of the determined first number of cycles and the determined second number of cycles. In some of these embodiments, the determined skin parameter is skin tone.

Therefore, there is provided an improved method and apparatus for determining a value for a skin parameter.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for determining a value of a skin parameter for skin of a subject, the method comprising:
receiving (510) a first sensor signal that is output by a first light sensor that is arranged to receive light emitted by a first light source after interaction of the emitted light with the skin, wherein the first sensor signal is output while the first light source operates according to a first duty cycle to intermittently emit the light, and wherein the emitted light has a first central wavelength;
receiving (520) a first modulation reference signal that is related to the first duty cycle;
integrating (530) a first function output signal, resulting from a function of the first sensor signal and the first modulation reference signal, to determine a first integrated value;
determining (540) a first number of cycles of the emitted light that are required for the first integrated value to exceed a first threshold value; and
determining (550) the value of the skin parameter based on the determined first number of cycles.

2. A method as claimed in claim 1, wherein the first modulation reference signal has a modulation period corresponding to a modulation period of the first duty cycle.

3. A method as claimed in claim 1, wherein the first modulation reference signal and the function of the first sensor signal and the first modulation reference signal result in the first function output signal corresponding to a first multiple of the first sensor signal for parts of the first duty cycle in which the first light source is emitting light, and corresponding to the inverse of the first multiple of the first sensor signal for parts of the first duty cycle in which the first light source is not emitting light.

4. A method as claimed in any of claims 1-3, wherein the first sensor signal is an analog signal output by the first light sensor and wherein the step of integrating (530) comprises integrating the first function output signal in the analog domain.

5. A method as claimed in claim 4, wherein the step of integrating (530) comprises inputting the first function output signal into one or more analog electronics integrators.

6. A method as claimed in claim 5, wherein the first integrated value is an electrical voltage across the one or more analog electronics integrators.

7. A method as claimed in any of claims 1-3, wherein the step of integrating (530) comprises integrating the first function output signal in the digital domain.

8. A method as claimed in any of claims 1-7, wherein the method further comprises:
receiving (510) a second sensor signal that is output by the first light sensor or a second light sensor that is arranged to receive light emitted by the first light source or a second light source after interaction of the emitted light with the skin, wherein the second sensor signal is output while the first light source or the second light source operates according to a second duty cycle to intermittently emit light having a second central wavelength different from the first central wavelength;
receiving (520) a second modulation reference signal that is related to the second duty cycle;
integrating (530) a second function output signal, resulting from a function of the second sensor signal and the second modulation reference signal, to determine a second integrated value; and
determining (540) a second number of cycles of the light having the second central wavelength that are required for the second integrated value to exceed a second threshold value;
wherein the step of determining (550) the value of the skin parameter comprises determining the value of the skin parameter based on a function of the determined first number of cycles and the determined second number of cycles.

9. A computer program product comprising a computer readable medium having computer readable code embodied therein, the computer readable code being configured such that, on execution by a suitable computer or processor, the computer or processor is caused to perform the method of any of claims 1-8.

10. An apparatus (42) for determining a value of a skin parameter for skin of a subject, the apparatus (42) being configured to:
receive a first sensor signal that is output by a first light sensor that is arranged to receive light emitted by a first light source after interaction of the emitted light with the skin, wherein the first sensor signal is output while the first light source operates according to a first duty cycle to intermittently emit the light, and wherein the emitted light has a first central wavelength;
receive a first modulation reference signal that is related to the first duty cycle;
integrate a first function output signal, resulting from a function of the first sensor signal and the first modulation reference signal to determine a first integrated value;
determine a first number of cycles of the emitted light that are required for the first integrated value to exceed a first threshold value; and
determine the value of the skin parameter based on the determined first number of cycles.

11. An apparatus (42) as claimed in claim 10, wherein the first modulation reference signal has a modulation period corresponding to a modulation period of the first duty cycle.

12. An apparatus (42) as claimed in claim 10, wherein the first modulation reference signal and the function of the first sensor signal and the first modulation reference signal result in the first function output signal corresponding to a first multiple of the first sensor signal for parts of the first duty cycle in which the first light source is emitting light, and corresponding to the inverse of the first multiple of the first sensor signal for parts of the first duty cycle in which the first light source is not emitting light.

13. An apparatus (42) as claimed in claim 10, 11 or 12, wherein the apparatus (42) comprises one or more analog electronics integrators and the apparatus (42) is configured to integrate the first function output signal by inputting the first function output signal into the one or more analog electronics integrators.

14. An apparatus (42) as claimed in any of claims 10-13, wherein the apparatus (42) is configured to integrate the first function output signal by integrating in the digital domain.

15. An apparatus (42) as claimed in any of claims 10-14, wherein the apparatus is further configured to: (i) receive a second sensor signal that is output by the first light sensor or a second light sensor that is arranged to receive light emitted by the first light source or a second light source after interaction of the emitted light with the skin, wherein the second sensor signal is output while the first light source or the second light source operates according to a second duty cycle to intermittently emit light having a second central wavelength different from the first central wavelength; (ii) receive a second modulation reference signal that is related to the second duty cycle; (iii) integrate a second function output signal, resulting from a function of the second sensor signal and the second modulation reference signal, to determine a second integrated value; and (iv) determine a second number of cycles of the light having the second central wavelength that are required for the second integrated value to exceed a second threshold value; wherein the apparatus is configured to determine the value of the skin parameter based on a function of the determined first number of cycles and the determined second number of cycles.

16. A system (40) comprising:
an apparatus (42) according to any of claims 9-15;
the first light source (44) configured to operate according to the first duty cycle to intermittently emit the light having the first central wavelength; and
the first light sensor (46) configured to output the first sensor signal.
